# EUROPEAN PATENT SPECIFICATION

(11) **EP 4 523 744 B1**
(45) Date of publication and mention of the grant of the patent: **16.09.2026**
(21) Application number: 24215203.1
(22) Date of filing: 24.09.2018
(51) Int. Cl.: A61K 31/192, A61K 9/00, A61P 17/04, A61P 1/16

(54) **COMPOSITION COMPRISING SELADELPAR FOR THE TREATMENT OF CHOLESTATIC PRURITUS**
SEDALPAR ENTHALTENDE ZUSAMMENSETZUNG ZUR BEHANDLUNG VON CHOLESTATISCHEM PRURITUS
COMPOSITION CONTENANT DU SÉDALPAR POUR LE TRAITEMENT DU PRURIT CHOLESTATIQUE

(30) Priority: 26.09.2017 US 201762563395 P
(43) Date of publication of application: 19.03.2025
(60) Divisional application: 26196734.3
(62) Document number(s) of the earlier application(s) in accordance with Art. 76 EPC: 21201888.1 / 3 973 959; 18811954.9 / 3 687 523
(73) Proprietor: CymaBay Therapeutics, Inc., Foster City, CA 94404 (US)
(72) Inventor: BOUDES, Pol, Pennington, 08534 (US); MCWHERTER, Charles A., Foster City, 94404 (US); STEINBERG, Alexandra S., Foster City, 94404 (US)
(74) Representative: Carpmaels & Ransford LLP

(56) References cited:
- WO-A1-2015/143178
- D JONES ET AL: "A phase 2 proof of concept study of MBX-8025 in patients with Primary Biliary Cholangitis (PBC) who are inadequate responders to ursodeoxycholic acid (UDCA)", CYMABAY THERAPEUTICS, February 2017 (2017-02-01), pages 1 - 16, XP055564310, Retrieved from the Internet <URL:https://content.equisolve.net/cymabay/media/2ab054a43f96c6154a9f5ce9f2355a4d.pdf> [retrieved on 20190304]
- ANONYMOUS: "Seladelpar (MBX-8025) in Subjects With Primary Biliary Cholangitis (PBC)", CLINICALTRIALS.GOV, 28 August 2017 (2017-08-28), pages 1 - 8, XP055564774, Retrieved from the Internet <URL:https://www.clinicaltrials.gov/ct2/history/NCT02955602?V_21=View#StudyPageTop> [retrieved on 20190305]
- ANONYMOUS: "Study to Evaluate the Effects of Two Doses of MBX-8025 in Subjects With Primary Biliary Cirrhosis (PBC)", CLINICALTRIALS.GOV, 30 January 2017 (2017-01-30), pages 1 - 10, XP055564778, Retrieved from the Internet <URL:https://www.clinicaltrials.gov/ct2/history/NCT02609048?V_31=View#StudyPageTop> [retrieved on 20190305]
- VINOD S HEGADE ET AL: "A systematic approach to the management of cholestatic pruritus in primary biliary cirrhosis", FRONTLINE GASTROENTEROLOGY, vol. 7, no. 3, 26 August 2015 (2015-08-26), pages 158 - 166, XP055564100, ISSN: 2041-4137, DOI: 10.1136/flgastro-2015-100618
- MARINA G. SILVEIRA ET AL: "Investigational drugs in phase II clinical trials for primary biliary cholangitis", EXPERT OPINION ON INVESTIGATIONAL DRUGS, vol. 26, no. 10, 24 August 2017 (2017-08-24), UK, pages 1115 - 1121, XP055523264, ISSN: 1354-3784, DOI: 10.1080/13543784.2017.1371135

## Description

### Technical field

This invention relates to the treatment of cholestatic pruritus.

### Background art

### Cholestatic pruritus

Pruritus (itch) is a well-known, frequent, and often distressing symptom of various hepatobiliary diseases, particularly cholestatic disorders, where it is referred to as cholestatic pruritus or pruritus of cholestasis. It may be mild and tolerable, but it may also dramatically reduce quality of life, cause severe sleep deprivation and depressive mood, and may even induce suicidal ideation in sufferers most affected by it. The great majority of sufferers report a diurnal variation in itch intensity, with the most intense itch in the late evening and early night. The itch is typically reported as localized at the limbs and the soles of the feet and palms of the hands (palmoplantar pruritus), but generalized itch may also occur. The itch is often exacerbated by psychological stress, heat, and contact with certain fabrics such as wool.

Beuers et al., "Pruritus in Cholestasis: Facts and Fiction", Hepatology, vol. 60, pp. 399-407 (2014), list in their Table 1 (page 401), the following examples of hepatobiliary diseases typically associated with cholestatic pruritus:
hepatocellular cholestasis:
intrahepatic cholestasis of pregnancy (ICP); estrogen-, progesterone- or testosterone-induced cholestasis; toxin- or other drug-induced hepatocellular cholestasis; benign recurrent intrahepatic cholestasis (BRIC); progressive familial intrahepatic cholestasis type 1 and 2 (PFIC1, PFIC2); and chronic viral hepatitis C (but they note that parenteral nutrition-induced cholestasis, chronic hepatitis B, and alcoholic or nonalcoholic fatty liver disease (NAFLD) are not or are only exceptionally accompanied by itch),
cholangiocellular cholestasis (with intrahepatic bile duct damage):
   primary biliary cholangitis (PBC - formerly called primary biliary cirrhosis); primary sclerosing cholangitis (PSC); secondary sclerosing cholangitis (SSC); sarcoidosis; ABCB4 deficiency (including PFIC3); Alagille syndrome (AS); and drug-induced small duct cholangiopathies (but they note that ductal plate malformations such as biliary hamartomas [von Meyenburg complexes], Caroli syndrome, and congenital liver fibrosis are typically not accompanied by itch),
   obstructive cholestasis:
      gallstone disease; primary and secondary sclerosing cholangitis (PSC, SSC); IgG4-associated cholangitis; biliary atresia; cholangiocellular carcinoma; benign bile duct adenoma; hilar lymphadenopathy; and pancreatic head carcinoma.

Hegade et al., "Drug treatment of pruritus in liver diseases", Clin. Med., vol. 15(4), pp. 351-357 (2015), say that "In clinical practice, the most commonly encountered cholestatic liver diseases (CLD) associated with pruritus are primary biliary cirrhosis (PBC), primary sclerosing cholangitis (PSC) and intrahepatic cholestasis of pregnancy. Historically, pruritus has been observed to accompany jaundice, but it is not uncommon to see pruritus as the first manifestation of cholestasis even before the onset of jaundice or other symptoms. There is considerable variation in the frequency and prevalence of pruritus in different cholestatic conditions. For example, it is experienced by up to 80% of patients with PBC and PSC and by 5-15% of patients with chronic hepatitis C at any time during the course of their disease. It is less common in patients with extrahepatic cholestasis, as in one series pruritus occurred in 17% of all patients with non-neoplastic obstructive jaundice and in 45% of patients with neoplastic obstructive jaundice. Also, pruritus is rare in common liver diseases such as alcohol-related liver diseases and non-alcohol fatty liver diseases. Interestingly, for reasons that are currently unexplained, the severity of pruritus seen in cholestatic conditions has no relationship with the degree of severity of cholestasis, i.e. patients with similar severities of liver disease and cholestasis can have markedly different degrees of pruritus." Hegade et al., "A systematic approach to the management of cholestatic pruritus in primary biliary cirrhosis", Frontline Gastroenterology, vol. 7, pp. 158-166 (2016), note that pruritus in patients with PBC is independent of the biochemical severity, duration of the disease, and histological stage of PBC; so that a patient with early-stage PBC and normal liver function tests may have severe itch while a patient with advanced PBC and liver dysfunction may have no pruritus.

According to Beuers et al., studies on signal transmission in pruritus have revealed that, contrary to the prevailing view of as recently as 20 years ago, itch perception is not transmitted by pain fibers, and that pain itself has an inhibitory effect on itch. A variety of molecules have been discussed as potential causative agents in cholestatic pruritus, including endogenous opioids, histamine, serotonin, various steroid metabolites (particularly progestogens and estrogens), and, most importantly, bile salts. But, according to Beuers et al., serum and/or tissue levels of these suspect pruritogens have never been found to closely correlate with itch intensity, making them less likely to be the true causative agent, though some might modulate rather than initiate the itch signaling chains *(see* Beuers et al. Table 2, page 403). Beuers et al. conclude that a key factor for the initiation of itch in cholestatic pruritus may be lysophosphatidic acid (LPA) and autotaxin (ATX, also known as lysophospholipase D, NPP2, and ENPP2), which is responsible for the formation of LPA from lysophosphatidylcholine; and they note observations supporting this, though they also note that ATX elevation can occur in noncholestatic physiological states such as normal pregnancies.

Pruritus may be numerically assessed in several ways. Two single-dimensional numerical assessment methods are the Visual Analog Scale (VAS), in which the subject is presented with a line with the left end-point labeled as "no itching" and the right end-point labeled as "worst possible itching", and the Numerical Rating Scale (NRS), in which the subject is presented with a line marked like a ruler, typically from 0 to 10 or 0 to 100, or as a series of 11 squares numbered from 0 to 10. In either method, the subject is asked to mark a place on the line, or select a square, corresponding to their level of itching, which may be their present level of itching, or the worst level of itching they have experienced in a recall period such as the previous 24 hours. The term VAS is sometimes used also to describe a scale where the line is marked like a ruler in addition to having the end-points labeled. The VAS has been validated for use in clinical trials for measuring pruritus and is recommended by the International Forum for the Study of Itch (Ständer et al., "Pruritus Assessment in Clinical trials: Consensus Recommendations from the International Forum for the Study of Itch (IFSI) Special Interest Group Scoring Itch in Clinical Trials", Acta Derm. Venereol., vol. 93, pp. 509-514 (2013)). Two multidimensional numerical assessment methods are the 5-D itch scale (Elman et al., "The 5-D itch scale: a new measure of pruritus", Br. J. Dermatol., vol. 162(3), pp. 587-593 (2010)), which assesses pruritus over the past two weeks on duration (how long per day), degree (how intense), direction (increasing or decreasing), disability (impact of the itching on life), and distribution (body location), with each dimension scored numerically; and the PBC-40 (Jacoby et al., "Development, validation, and evaluation of the PBC-40, a disease specific health related quality of life measure for primary biliary cirrhosis", Gut, vol. 54, pp. 1622-1629 (2005)), which uses 40 questions about quality of life over the past four weeks, with 3 numerically-scored questions specific to itching.

### Pharmacological treatments for cholestatic pruritus

Hegade et al. (Clin. Med.) list the following as evidence-based therapeutic recommendations for cholestatic pruritus (Table 2, page 353):
the bile acid resins cholestyramine and colesevelam, anion exchange resins used for bile acid absorption, recommended as first-line therapy. Side effects include unpleasant taste, bloating, constipation, and diarrhea; and interactions (generally, reduced absorption) with oral diabetes drugs, thiazide diuretics, warfarin, and others, requiring that they be given 2-4 hours before or after other medications). Hegade et al. note that while colesevelam causes fewer side effects than cholestyramine, a small double-blind trial found it no more effective than placebo in relieving cholestatic pruritus in PBC and PSC;
rifampicin, a common antibacterial agent that is a pregnane X receptor agonist and enzyme inducer, recommended as second-line therapy. Rifampicin has been shown to reduce serum ATX levels compared with placebo, and treatment leads to at least partial resolution of pruritus in many patients. However, side effects include nausea, vomiting, diarrhea, decreased appetite, headache, fever, rash, and flushing, though these are all typically transient and resolve on discontinuation; while more serious side effects include hepatitis, hemolytic anemia, thrombocytopenia, renal impairment, and alteration in drug metabolism: an early study reported a 12.5% incidence of rifampicin hepatitis, and a more recent study reported a 7.3% incidence of significant hepatitis. Blood count and liver biochemistry monitoring is required;
naltrexone, an opioid antagonist, recommended as third-line therapy. Several studies show that oral antagonists such as naltrexone and nalmefene, and the IV antagonist naloxone, are all more likely to reduce cholestatic pruritus than the control intervention. However, these agents have the significant concern of opioid withdrawal-like reaction - a group of symptoms characterized by abdominal pain, tachycardia, high blood pressure, goose bumps, nightmares, and depersonalization - though this reaction may be minimized by upward dose titration.
Hepatotoxicity has also been reported, and the opioid antagonists are contraindicated in acute hepatitis, liver failure, suppressed pulmonary function, drug addiction, and in those receiving opioid medications; and
sertraline, a selective serotonin reuptake inhibitor, commonly prescribed as an antidepressant, recommended as fourth-line therapy. Hegade et al. report that two studies have shown that sertraline was well tolerated and moderately effective in reducing the intensity of itch in cholestatic pruritus, with an effect independent from improvement in depression. Sertraline is generally well-tolerated, but uncommon side effects include nausea, dizziness, diarrhea, visual hallucinations, and increased fatigue.

Experimental agents include:
ASBT (apical sodium-dependent bile acid transporter - also known as IBAT: ileal bile acid transporter) inhibitors. GSK2330672, a selective inhibitor of human ASBT, completed a Phase 2a study in PBC patients with pruritus in 2016; and it was reported that GSK2330672 produced significantly greater reductions in itch scores (1-10 numerical rating scale, PBC-40 itch domain score, and 5-D itch scale) than placebo. The most common side effect was diarrhea (33% of subjects), which, the investigators say, "might limit the long-term use of this drug". A larger dose-response study (NCT02966834) is recruiting;
fibrates (fenofibrate and bezafibrate), already considered as anticholestatic therapy for PBC in combination with UDCA. Early Japanese studies had reported improvement or disappearance of pruritus in patients with PBC treated with fibrates. A two-year Phase 3 study (BEZURSO, NCT01654731) in PBC patients with an inadequate biochemical response to ursodeoxycholic acid (UDCA, ursodiol), adding 400 mg/day of bezafibrate or placebo to the patient's standard dose of ursodiol, was reported at the International Liver Congress in April 2017 (Corpechot et al., "A 2-year multicenter, double-blind, placebo-controlled study of bezafibrate for the treatment of primary biliary cholangitis in patients with inadequate response to ursodeoxycholic acid (Bezurso)", J. Hepatol., vol. 66, p. S89, Abstract LBO-01 (2017)), and published in 2018 (Corpechot et al., "A Placebo-Controlled Trial of Bezafibrate in Primary Biliary Cholangitis", New Engl. J. Med., vol. 378(23), pp. 2171-2181 (2018)). In addition to improvements in liver function scores, itch scores assessed by VAS decreased in the bezafibrate group; though the significance of the of that result has been criticized in subsequent correspondence. A three-week Phase 3 study (FITCH, NCT02701166), using 400 mg/day of bezafibrate or placebo in patients with PBC, PSC, or SSC, and an itch score of ≥ 5.0 on a scale from 0.0 (no itch) to 10.0 (worst itch possible), with a primary endpoint of reduction in itch of 50% or more, was recruiting in Europe, but its present status is unknown; and
ATX/LPA inhibitors. Castagna et al., "Development of Autotaxin Inhibitors: An Overview of the Patent and Primary Literature", J. Med. Chem., vol. 59, pp. 5604-5621 (2016), report that although ATX was first isolated in 1992, only one ATX inhibitor has progressed to clinical trials. That compound, GLPG1690, has completed a twelve-week placebo-controlled Phase 2a study (NCT02738801) in idiopathic pulmonary fibrosis.

UDCA itself has been shown in several trials to alleviate itch and improve serum liver tests in intrahepatic cholestasis of pregnancy, where it is regarded as first-line treatment. UDCA is a common treatment for intrahepatic cholestatic diseases, because of its action in reducing cholestasis by improving hepatobiliary secretion; but it is not effective in reducing pruritus in other forms of cholestatic pruritus, and current guidelines do not recommend its use.

Obeticholic acid (OCA, 6α-ethylchenodeoxycholic acid, Intercept Pharmaceuticals's OCALIVA), a semi-synthetic bile acid analog that is a highly potent farnesoid X receptor agonist, is approved in the US for the treatment of PBC in conjunction with UDCA or where UDCA is not tolerated. However, while it lowers alkaline phosphatase (a biomarker of cholestasis), it has the common side effect of exacerbating pruritus. From the OCALIVA prescribing information, severe pruritus was reported in 23% of patients in the OCALIVA 10 mg arm, 19% of patients in the OCALIVA titration arm, and 7% of patients in the placebo arm in a 12-month double-blind randomized controlled trial of 216 patients.

Thus, anti-cholestatic agents such as the bile acid analogs may have no effect, or even a worsening effect, on cholestatic pruritus.

### Seladelpar

Seladelpar (International Nonproprietary Name - INN) has the chemical name [4-({*(2R)*-2-ethoxy-3-[4-(trifluoromethyl)phenoxy]propyl}sulfanyl)-2-methylphenoxy]acetic acid [IUPAC name from WHO Recommended INN: List 77], and the code number MBX-8025. Seladelpar and its synthesis, formulation, and use is disclosed in, for example, US Patent No. 7301050 (compound 15 in Table 1, Example M, claim 49), US Patent No. 7635718 (compound 15 in Table 1, Example M), and US Patent No. 8106095 (compound 15 in Table 1, Example M, claim 14). Lysine (L-lysine) salts of seladelpar and related compounds are disclosed in US Patent No. 7709682 (seladelpar L-lysine salt throughout the Examples, crystalline forms claimed).

Seladelpar is an orally active, potent (2 nM) agonist of peroxisome proliferator-activated receptor-δ (PPARδ). It is specific (>600-fold and >2500-fold relative to PPARα and PPARγ). PPARδ activation stimulates fatty acid oxidation and utilization, improves plasma lipid and lipoprotein metabolism, glucose utilization, and mitochondrial respiration, and preserves stem cell homeostasis. According to US Patent No. 7301050, PPARδ agonists, such as seladelpar, are suggested to treat PPARδ-mediated conditions, including "diabetes, cardiovascular diseases, Metabolic X syndrome, hypercholesterolemia, hypo-high density lipoprotein (HDL)-cholesterolemia, hyper-low density protein (LDL)-cholesterolemia, dyslipidemia, atherosclerosis, and obesity", with dyslipidemia said to include hypertriglyceridemia and mixed hyperlipidemia.

US Patent No. 9486428 and PCT International Publication No. WO 2015/143178 disclose the treatment of intrahepatic cholestatic diseases, such as PBC, PSC, PFIC, and AS, with seladelpar and its salts.

D Jones ET AL: "A phase 2 proof of concept study of MBX-8025 in patients with Primary Biliary Cholangitis (PBC) who are inadequate responders to ursodeoxycholic acid (UDCA)", CymaBay Therapeutics, February 2017, disclose the results of the treatment of PBC with seladelpar in patients not responding to UDCA.

### Summary of the invention

This invention relates to the treatment of cholestatic pruritus associated with primary biliary cholangitis by administration of a pharmaceutical composition comprising seladelpar or a salt thereof.

The invention is:
A pharmaceutical composition for use in treating cholestatic pruritus, wherein the cholestatic pruritus is associated with primary biliary cholangitis (PBC), wherein the composition comprises seladelpar or a salt thereof.

Seladelpar has already been demonstrated to be effective in the treatment of PBC at oral doses of 5, 10, 50, and 200 mg/day; and is expected to be effective in dosages between 0.5 mg/day and 25 mg/day. It is expected to be useful in other intrahepatic cholestatic diseases at similar dosages; and is also expected to be useful in the treatment of cholestatic pruritus.

Preferred embodiments of this invention are characterized by the specification and by the features of Claims 1 to 7 of this application.

### Description of the invention

### Definitions

Cholestatic pruritus and its treatment are described in the subsections entitled "Cholestatic pruritus" and "Treatments for cholestatic pruritus" of the Background art.

"Treating" or "treatment" of cholestatic pruritus in a human includes one or more of:
(1) preventing or reducing the risk of developing pruritus, i.e., causing the cholestatic pruritus not to develop in a subject who may be predisposed to an condition for which cholestatic pruritus is a symptom but who does not yet experience or display the pruritus (i.e. prophylaxis);
(2) inhibiting the pruritus, i.e., arresting or reducing the development of the pruritus; and
(3) relieving the pruritus, i.e., reducing the number, frequency, duration or severity of the pruritus.

A "therapeutically effective amount" of seladelpar or a seladelpar salt means that amount which, when administered to a human for treating cholestatic pruritus, is sufficient to effect treatment for the pruritus. The therapeutically effective amount for a particular subject varies depending upon the age, health and physical condition of the subject to be treated, the underlying hepatobiliary disease, the pruritus and its extent, the assessment of the medical situation, and other relevant factors. It is expected that the therapeutically effective amount will fall in a relatively broad range that can be determined through routine trial.

Seladelpar is described in the subsection entitled "Seladelpar" of the Background art.

Salts (for example, pharmaceutically acceptable salts) of seladelpar are included in this invention and are useful in the methods described in this application. These salts are preferably formed with pharmaceutically acceptable acids. See, for example, "Handbook of Pharmaceutically Acceptable Salts", Stahl and Wermuth, eds., Verlag Helvetica Chimica Acta, Zürich, Switzerland, for an extensive discussion of pharmaceutical salts, their selection, preparation, and use. Unless the context requires otherwise, reference to seladelpar is a reference both to the compound and to its salts.

Because seladelpar contains a carboxyl group, it may form salts when the acidic proton present reacts with inorganic or organic bases. Typically seladelpar is treated with an excess of an alkaline reagent, such as hydroxide, carbonate or alkoxide, containing an appropriate cation. Cations such as Na⁺, K⁺, Ca²⁺, Mg²⁺, and NH₄⁺ are examples of cations present in pharmaceutically acceptable salts. Suitable inorganic bases, therefore, include calcium hydroxide, potassium hydroxide, sodium carbonate and sodium hydroxide. Salts may also be prepared using organic bases, such as salts of primary, secondary and tertiary amines, substituted amines including naturally-occurring substituted amines, and cyclic amines, including isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, N-ethylpiperidine, and the like. As noted in the "Seladelpar" subsection, seladelpar is currently formulated as its L-lysine dihydrate salt.

"Comprising" or "containing" and their grammatical variants are words of inclusion and not of limitation and mean to specify the presence of stated components, groups, steps, and the like but not to exclude the presence or addition of other components, groups, steps, and the like. Thus "comprising" does not mean "consisting of", "consisting substantially of", or "consisting only of"; and, for example, a formulation "comprising" a compound must contain that compound but also may contain other active ingredients and/or excipients.

### Formulation and administration

Seladelpar may be administered by any route suitable to the subject being treated and the nature of the subject's condition. Routes of administration include administration by injection, including intravenous, intraperitoneal, intramuscular, and subcutaneous injection, by transmucosal or transdermal delivery, through topical applications, nasal spray, suppository and the like or may be administered orally. Formulations may optionally be liposomal formulations, emulsions, formulations designed to administer the drug across mucosal membranes or transdermal formulations. Suitable formulations for each of these methods of administration may be found, for example, in "Remington: The Science and Practice of Pharmacy", 20th ed., Gennaro, ed., Lippincott Williams & Wilkins, Philadelphia, Pa., U.S.A. Because seladelpar is orally available, typical formulations will be oral, and typical dosage forms will be tablets or capsules for oral administration. As mentioned in the "Seladelpar" subsection, seladelpar has been formulated in capsules for clinical trials.

Depending on the intended mode of administration, the pharmaceutical compositions may be in the form of solid, semi-solid or liquid dosage forms, preferably in unit dosage form suitable for single administration of a precise dosage. In addition to an effective amount of seladelpar, the compositions may contain suitable pharmaceutically-acceptable excipients, including adjuvants which facilitate processing of the active compounds into preparations which can be used pharmaceutically. "Pharmaceutically acceptable excipient" refers to an excipient or mixture of excipients which does not interfere with the effectiveness of the biological activity of the active compound(s) and which is not toxic or otherwise undesirable to the subject to which it is administered.

For solid compositions, conventional excipients include, for example, pharmaceutical grades of mannitol, lactose, starch, magnesium stearate, sodium saccharin, talc, cellulose, glucose, sucrose, magnesium carbonate, and the like. Liquid pharmacologically administrable compositions can, for example, be prepared by dissolving, dispersing, etc., an active compound as described herein and optional pharmaceutical adjuvants in water or an aqueous excipient, such as, for example, water, saline, aqueous dextrose, and the like, to form a solution or suspension. If desired, the pharmaceutical composition to be administered may also contain minor amounts of nontoxic auxiliary excipients such as wetting or emulsifying agents, pH buffering agents and the like, for example, sodium acetate, sorbitan monolaurate, triethanolamine sodium acetate, triethanolamine oleate, etc.

For oral administration, the composition will generally take the form of a tablet or capsule; or, especially for pediatric use, it may be an aqueous or nonaqueous solution, suspension or syrup. Tablets and capsules are preferred oral administration forms. Tablets and capsules for oral use will generally include one or more commonly used excipients such as lactose and corn starch. Lubricating agents, such as magnesium stearate, are also typically added. When liquid suspensions are used, the active agent may be combined with emulsifying and suspending excipients. If desired, flavoring, coloring and/or sweetening agents may be added as well. Other optional excipients for incorporation into an oral formulation include preservatives, suspending agents, thickening agents, and the like.

Typically, a pharmaceutical composition of seladelpar, or a kit comprising compositions of seladelpar, is packaged in a container with a label, or instructions, or both, indicating use of the pharmaceutical composition or kit in the treatment of cholestatic pruritus.

A suitable (i.e. a therapeutically effective) amount of seladelpar or a salt thereof for oral dosing, when the amount is calculated as seladelpar, is expected to be at least 0.5 mg/day, for example at least 1 mg/day, such as at least 2 mg/day, or at least 5 mg/day; but not more than 50 mg/day, for example not more than 25 mg/day, such as not more than 15 mg/day, or not more than 10 mg/day; for example within any range defined by one of the "at least" values and one of the "not more than" values, such as at least 1 mg/day and not more than 25 mg/day (i.e. 1 - 25 mg/day) or at least 2 mg/day and not more than 10 mg/day; for example 2 mg/day, 5 mg/day, or 10 mg/day, for an adult subject with cholestatic pruritus, depending on the extent and severity of the pruritus, the underlying condition associated with the pruritus, and factors such as hepatic and renal function. That is, a suitable amount of seladelpar for oral dosing for adults to treat cholestatic pruritus in conditions such as PBC is expected to be below the low end of the amounts employed in Example 1 but include the amounts employed in Example 2. Suitable reductions in dose toward or below the lower end of the outer range above will be made for subjects who are children in diseases such as PFIC and AS, depending on such additional factors as age and body mass; and in subjects with significant hepatic impairment, such as subjects in Child-Pugh classes B and C, depending on the degree of impairment. These amounts represent an average daily dose, and not necessarily an amount given at a single dose. Dosing may be as frequent as more than once/day (where the amount, or daily dose, will be divided between the number of administrations per day), but will more typically be once/day (where the amount is given in a single administration). Optionally, particularly in cases of significant hepatic impairment, the dosing may be less frequent than once/day, such as between once/week and every other day, for example once/week, twice/week (especially with the doses at least three days apart), three times/week (especially with the doses at least two days apart), or every other day; so that, as an example, a subject may receive 5 mg twice/week for an amount (daily dose) of 1.4 mg/day.

A person of ordinary skill in the art of the treatment of cholestatic pruritus, who will typically be a person of ordinary skill in the art of the treatment of hepatobiliary diseases, but may be a gynecologist in the case of ICP, will be able to ascertain a therapeutically effective amount of seladelpar or a seladelpar salt for a particular extent of pruritus, underlying hepatobiliary disease, and patient to achieve a therapeutically effective amount for the treatment of cholestatic pruritus without undue experimentation and in reliance upon personal knowledge, the skill of the art, and the disclosure of this application.

### Examples

### Example 1: High dose trial in PBC (NCT02609048)

The trial subjects were adult, male or female, with a diagnosis of PBC by at least two of the following three criteria: (a) a history of alkaline phosphatase (ALP) above the upper limit of normal (ULN) for at least six months, (b) positive anti-mitochondrial antibody titers > 1/40 on immunofluorescence or M2 positive by enzyme linked immunosorbent assay or positive PBC-specific antinuclear antibodies, and (c) documented liver biopsy result consistent with PBC, on a stable and recommended dose of UDCA for the past twelve months, and ALP ≥ 1.67 × ULN. Exclusion criteria included AST or ALT ≥ 3 × ULN, total bilirubin (TBIL) ≥ 2 × ULN, autoimmune hepatitis or a history of chronic viral hepatitis, PSC, the current use of fibrates or simvastatin, the use of colchicine, methotrexate, azathioprine, or systemic steroids in the previous two months, the use of an experimental treatment for PBC, and the use of an experimental or unapproved immunosuppressant. Subjects were randomized to receive either placebo, 50 mg/day, or 200 mg/day of seladelpar as the L-lysine dihydrate salt orally in capsule form, once/day dosing, for 12 weeks. Pruritus was assessed using VAS, 5-D itch, and PBC-40. During the study, three cases of asymptomatic increases in transaminases were observed (two in the 200 mg and one in the 50 mg groups): all were reversible on cessation of treatment and were not accompanied by elevation of total bilirubin. Since the study had already shown a clear efficacy signal, the study was discontinued. After the study was unblinded, changes in the primary endpoint ALP were analyzed using data available for the 26 subjects (10 in the placebo group, 9 in the 50 mg/day seladelpar group, and 7 in the 200 mg/day seladelpar group) enrolled in the study and completing at least two weeks of treatment; and a potent anti-cholestatic effect was observed. Despite the potent anti-cholestatic effect, no adverse events of pruritus were reported on treatment.

### Example 2: Low-dose trial in PBC (NCT02955602)

This example describes a study like that of Example 1, but including subjects who were intolerant of UDCA, and using doses of 5 mg/day or 10 mg/day of seladelpar as the L-lysine dihydrate salt orally in capsule form, once/day dosing. At 12 weeks, seladelpar even in doses as low as 5 mg/day reversed cholestasis, decreased transaminases, decreased LDL-cholesterol, and reduced inflammation. One subject entering the study with intense pruritus discontinued after five days of seladelpar at 10 mg/day for increased pruritus possibly related to PBC.

At 12 weeks, the results of pruritus assessment by VAS (0-100 scale) were as follows:

| | Seladelpar 5 mg/day | | | | Seladelpar 10 mg/day | | | |
|---|---|---|---|---|---|---|---|---|
| Visit | No. | Median | Q1 | Q3 | No. | Median | Q1 | Q3 |
| Visit 2 (Day 1) | 11 | 8 | 0 | 20 | 11 | 25 | 5 | 65 |
| Visit 3 (Week 1) | 11 | 10 | 2 | 30 | 10 | 13 | 4 | 50 |
| Visit 4 (Week 2) | 11 | 9 | 0 | 13 | 11 | 30 | 5 | 50 |
| Visit 5 (Week 4) | 11 | 10 | 0 | 15 | 11 | 20 | 5 | 33 |
| Visit 6 (Week 6) | 11 | 5 | 0 | 15 | 11 | 10 | 5 | 25 |
| Visit 7 (Week 8) | 11 | 7 | 0 | 25 | 11 | 15 | 3 | 30 |
| Visit 8 (Week 12) | 11 | 3 | 0 | 15 | 11 | 6 | 0 | 39 |

| | | | | | | | | |
|---|---|---|---|---|---|---|---|---|
| (Q1 = first/lower quartile, Q3 = third/upper quartile) | | | | | | | | |

As can be seen from the table, seladelpar at both 5 mg/day and 10 mg/day significantly reduced the cholestatic pruritus associated with PBC.

After 12 weeks, subjects in the 5 mg/day group were allowed to increase their seladelpar dose to 10 mg/day based on their ALP response (the 5/10 mg group). At 26 weeks, 119 subjects had received at least one dose of seladelpar, of whom 79 (66%) reported a history of pruritus. 37 subjects with VAS > 0 at baseline were analysed in the interim analysis at 26 weeks: 18 subjects in the 5/10 mg group and 19 in the 10 mg group. Baseline median VAS were 15 (range 1-68) and 50 (range 5-90) in the 5/10 mg and 10 mg groups, respectively. At week 26, median changes in VAS were -50% and -55% in the 5/10 mg and 10 mg groups, respectively. There were no serious adverse events due to pruritus; although pruritus was reported as an adverse event in 24/119 (20%) subjects. At 26 weeks, there had been no discontinuations related to seladelpar.

### Example 3

Adult subjects with a hepatobiliary disease typically associated with pruritus such as PSC, PFIC, or AS, are treated orally with doses of 1, 2, 5, and 10 mg/day of seladelpar. Subjects are permitted their usual other medications, including UDCA. The subjects are assessed before the study, and at intervals during the study, such as every 4 weeks during the study and 4 weeks after the last dose of the seladelpar therapy, for safety and pharmacodynamic evaluations. At each visit, the subjects are assessed for cholestatic symptoms and biomarkers, and assessed for cholestatic pruritus. The subjects also maintain health diaries, which are reviewed at each visit. The subjects show an improvement in their disease, as manifested by, for example, a decrease in ALP and GGT; and also an improvement in cholestatic pruritus.

## Claims

1. A pharmaceutical composition for use in treating cholestatic pruritus, wherein the cholestatic pruritus is associated with primary biliary cholangitis (PBC), wherein the composition comprises seladelpar or a salt thereof.

2. The composition for use according to claim 1, wherein the seladelpar or a salt thereof is seladelpar L-lysine salt.

3. The composition for use according to claim 1, wherein the seladelpar or a salt thereof is seladelpar L-lysine dihydrate salt.

4. The composition for use according to any one of claims 1 to 3, wherein the composition is administered orally.

5. The composition for use according to any one of claims 1 to 4, wherein the composition is administered once per day.

6. The composition for use according to any one of claims 1 to 5, wherein the amount of seladelpar or the salt thereof administered is 5 mg/day, wherein the amount is calculated as seladelpar.

7. The composition for use according to any one of claims 1 to 5, wherein the amount of seladelpar or the salt thereof administered is 10 mg/day, wherein the amount is calculated as seladelpar.

## Patentansprüche

1. Pharmazeutische Zusammensetzung zur Verwendung bei der Behandlung von cholestatischem Pruritus, wobei der cholestatische Pruritus mit primärer biliärer Cholangitis (PBC) assoziiert ist, wobei die Zusammensetzung Seladelpar oder ein Salz davon umfasst.

2. Zusammensetzung zur Verwendung nach Anspruch 1, wobei das Seladelpar oder ein Salz davon Seladelpar-L-Lysinsalz ist.

3. Zusammensetzung zur Verwendung nach Anspruch 1, wobei das Seladelpar oder ein Salz davon Seladelpar-L-Lysindihydratsalz ist.

4. Zusammensetzung zur Verwendung nach einem der Ansprüche 1 bis 3, wobei die Zusammensetzung oral verabreicht wird.

5. Zusammensetzung zur Verwendung nach einem der Ansprüche 1 bis 4, wobei die Zusammensetzung einmal pro Tag verabreicht wird.

6. Zusammensetzung zur Verwendung nach einem der Ansprüche 1 bis 5, wobei die verabreichte Menge an Seladelpar oder dem Salz davon 5 mg/Tag beträgt, wobei die Menge als Seladelpar berechnet wird.

7. Zusammensetzung zur Verwendung nach einem der Ansprüche 1 bis 5, wobei die verabreichte Menge an Seladelpar oder dem Salz davon 10 mg/Tag beträgt, wobei die Menge als Seladelpar berechnet wird.

## Revendications

1. Composition pharmaceutique à utiliser dans le traitement du prurit cholestatique, le prurit cholestatique étant associé à une cholangite biliaire primitive (CBP), la composition comprenant du séladelpar ou un sel de celui-ci.

2. Composition à utiliser selon la revendication 1, dans laquelle le séladelpar ou un sel de celui-ci est le sel de L-lysine de séladelpar.

3. Composition à utiliser selon la revendication 1, dans laquelle le séladelpar ou un sel de celui-ci est le sel de L-lysine de séladelpar dihydraté.

4. Composition à utiliser selon l'une quelconque des revendications 1 à 3, la composition étant administrée par voie orale.

5. Composition à utiliser selon l'une quelconque des revendications 1 à 4, la composition étant administrée une fois par jour.

6. Composition à utiliser selon l'une quelconque des revendications 1 à 5, la quantité administrée de séladelpar ou du sel de celui-ci étant de 5 mg/jour, la quantité étant calculée comme celle du séladelpar.

7. Composition à utiliser selon l'une quelconque des revendications 1 à 5, la quantité administrée de séladelpar ou du sel de celui-ci étant de 10 mg/jour, la quantité étant calculée comme celle du séladelpar.
